Europäisches Patentamt

(19) European Patent Office

Office européen des brevets

(11) EP 0 486 350 B1

(12) **FASCICULE DE BREVET EUROPEEN**

(45) Date de publication et mention
de la délivrance du brevet:
**25.03.1998 Bulletin 1998/13**

(51) Int. Cl.$^6$: **C07C 55/07**, C07C 51/41,
C01F 17/00

(21) Numéro de dépôt: **91402879.0**

(22) Date de dépôt: **28.10.1991**

(54) **Procédé de fabrication d'oxalates doubles de terres rares et d'ammonium et leurs utilisations pour la fabrication d'oxydes de terres rares et oxydes de terres rares obtenus**

Verfahren zur Herstellung von gemischten Oxalaten von Ammonium und seltenen Erden und deren Anwendung zur Herstellung von Oxyden der seltenen Erden und die so hergestellten seltenen Erden

Method for the production of mixed ammonium-rare earth oxalates and their application to the production of rare earth oxides and rare earth oxides so produced

(84) Etats contractants désignés:
**AT BE CH DE DK ES FR GB GR IT LI LU NL SE**

(30) Priorité: **13.11.1990 FR 9014030**

(43) Date de publication de la demande:
**20.05.1992 Bulletin 1992/21**

(73) Titulaire: **RHONE-POULENC CHIMIE**
**92408 Courbevoie Cédex (FR)**

(72) Inventeur: **David, Claire**
**F-75008 Paris (FR)**

(74) Mandataire:
**Dutruc-Rosset, Marie-Claude et al**
**RHONE-POULENC CHIMIE,**
**Direction de la Propriété Industrielle,**
**25, Quai Paul Doumer**
**92408 Courbevoie Cédex (FR)**

(56) Documents cités:
**BE-A- 748 348**

• **BULLETIN OF THE CHEMICAL SOCIETY OF JAPAN. vol. 63, no. 2, 1990, TOKYO JP pages 378 - 382; Y. Minagawa: "New preparative method of fine powder of Yttrium [III] oxide by thermal decomposition..."**

Printed by Xerox (UK) Business Services
2.15.12/3.4

## Description

La présente invention concerne un procédé de fabrication d'oxalates doubles de terres rares et d'ammonium, leur utilisation pour l'obtention d'oxyde de terres rares.

Elle se rapporte plus particulièrement à un procédé permettant d'obtenir des oxalates doubles présentant une morphologie et une granulométrie déterminées par transformation d'un oxalate neutre de terres rares.

Les oxydes de terres trouvent de nombreuses applications dans le domaine notamment de la céramique et de l'électronique mais à l'heure actuelle, on constate sur le marché une demande croissante de produits à granulométrie contrôlée.

Une des voies classiques pour obtenir des oxydes de terres rares et qui est largement décrite dans la littérature, notamment dans le NOUVEAU TRAITE DE CHIMIE MINERALE, Tome VII, (1959), P. 1007 de Paul PASCAL, consiste à calciner entre 500 et 900°C les oxalates de terres rares obtenus par précipitation à l'aide de l'acide oxalique, des sels de terres rares sous forme de solution aqueuse. Cependant, un tel procédé de fabrication ne conduit qu'à des oxydes de terres rares présentant une granulométrie comprise entre 3 et 6 μm.

Il est également connu selon JP 53 095911-A (Chemical Abstracts 90, 40940 w) de préparer des oxydes de terres rares fins et plus particulièrement de l'oxyde d'yttrium fin par calcination d'un oxalate d'yttrium et d'ammonium gui consiste à partir d'une solution aqueuse d'un sel d'yttrium, à précipiter l'yttrium sous la forme de son hydroxyde préparé par réaction de la solution aqueuse d'un sel d'yttrium et d'une solution aqueuse basique comme l'ammoniaque puis à traiter la bouillie d'hydroxyde résultante par l'acide oxalique, et enfin à séparer le précipité obtenu, à le laver et à le calciner à une température de 750°C. Ledit procédé conduit conformément à la description donnée dans JP 53-095911-A à l'obtention d'oxyde d'yttrium fin. Le diamètre des particules est compris entre 0,9 et 4,5 μm, les cristaux ayant une forme de plaquettes à bords arrondis.

Les deux articles du Bull. Chem. Soc. Japan 1990 63, 2115 et 63, 378 décrivent des méthodes de préparation de poudres d'oxyde d'yttrium par calcination de précipités qui sont obtenus respectivement par réaction de nitrate d'yttrium et d'ammoniaque, puis addition d'acide oxalique et par réaction d'ammoniaque avec une solution d'oxalate d'yttrium. Dans ces deux cas, on obtient des produits dont la taille moyenne est d'au plus 1 μm.

L'article du J. Inorg. Nucl. Chem. 1964, vol. 26, pp. 931-936 décrit des méthodes de précipitation analogues par ajout d'oxalate d'ammonium à une solution de nitrate d'yttrium ou d'ammoniaque à des solutions acides de nitrate.

Le Gmelin Handbook of Inorganic Chemistry (1984),part D5,System n° 39, pp. 141, 145 fait état d'oxalates doubles préparés par addition d'acide oxalique, puis de $NH_3$ à des solutions de nitrates de terres rares.

Toutefois dans certaines applications, des oxydes de terres rares comme l'oxyde d'yttrium présentant des dimensions de grains plus élevées sont demandés. De telles dimensions ne peuvent être obtenues avec les oxalates neutres de terrres rares utilisés comme précurseur.

Un des buts de la présente invention est notamment de remédier à ces inconvénients en proposant un procédé de fabrication d'oxalates doubles d'ammonium et de terres rares présentant des dimensions de grains élevées comprises, par exemple, entre 5 et 10 μm.

Par oxalate double de terres rares et d'ammonium, il faut comprendre un composé comprenant une ou plusieurs terres rares associées avec des ions ammonium et oxalates permettant après calcination de produire des oxydes simples ou mixtes.

Par l'expression terres rares il faut comprendre les éléments portant les numéros atomiques compris entre 57 et 71 (bornes incluses) appartenant à la famille des lanthanides, ainsi que l'yttrium portant le numéro atomique 39.

A cet effet, l'invention propose un procédé de fabrication d'un oxalate double de terres rares et d'ammonium caractérisé en ce qu'il consiste :

- a mélanger, en milieu aqueux, au moins un oxalate neutre de terres rares avec un composé capable de libérer des ions ammonium et un composé capable de libérer des ions oxalates en solution
- à séparer le précipité obtenu
- et, éventuellement le sécher.

Par oxalate neutre de terres rares, on entend un sel dans lequel toutes les fonctions acides de l'acide oxalique ont été neutralisées par le ou les cations terres rares.

Selon une caractéristique de l'invention la quantité d'ions oxalates ajoutés est suffisante pour avoir en fin de précipitation un rapport molaire des ions oxalates aux ions de terres rares avantageusement supérieur ou égal à 2. En effet, le rendement de la transformation de l'oxalate neutre en oxalate double sera plus élevé et égal à 100% si le rapport $C_2O_4^=/TR$ est élevé et notamment supérieur à 2.

Les composés capables de libérer des ions ammonium en solution convenables pour l'invention sont tous les composés solubles ou non dans l'eau qui mis en présence d'eau libèrent des ions ammonium.

On peut citer à titre d'exemple non limitatif, le nitrate d'ammonium, le chlorure d'ammonium, l'acétate d'ammonium, l'hydroxyde d'ammonium ou analogue.

De manière analogue, les composés capables de libérer des ions oxalates convenables pour l'invention sont tous les composés solubles ou non dans l'eau qui mis en présence d'eau libèrent des ions oxalates.

On peut citer à titre d'exemple non limitatif, l'acide oxalique cristallisé ou en solution, les oxalates d'alcalins ou analogue.

Dans un des modes de réalisation de l'invention, l'oxalate d'ammonium est utilisé comme composé pour l'apport simultané des ions ammonium et oxalate. Toutefois, il est possible d'utiliser cet oxalate d'ammonium en mélange avec un composé apportant des ions ammonium comme l'ammoniac, par exemple, ou un composé apportant des ions oxalates comme, par exemple, l'acide oxalique.

Selon une autre caractéristique de l'invention, les quantités de composés capables de libérer des ions oxalates et/ou des ions ammonium ajoutés dans le milieu contenant l'oxalate neutre de terres rares sont déterminées pour avoir en fin de précipitation des rapports molaires $C_2O_4^=$/TR et $NH_4^+$/TR supérieurs ou égaux à 2, de préférence supérieurs ou égaux à 2,5.

Les oxalates neutres de terres rares convenant avantageusement pour l'invention sont l'oxalate d'yttrium, europium, lanthane, néodyme, dysprosium, cérium, gadolinium, terbium ou un mélange de ceux-ci.

Bien que le procédé de l'invention s'applique tout-à-fait bien aux terres rares cériques, il convient plus particulièrement aux terres rares yttriques.

On entend par "terres rares cériques", les éléments les plus légers des terres rares commençant avec le lanthane et s'étendant jusqu'au néodyme conformément au numéro atomique et l'on désigne par "terres rares yttriques" les éléments les plus lourds des terres rares conformément au numéro atomique, commençant avec le samarium et finissant avec le lutécium et comprenant l'yttrium.

La concentration en composé de terres rares n'est pas critique.

Les oxalates neutres de terres rares sont obtenus par tous procédés connus et classiques comme par exemple, par précipitation de l'oxalate de terres rares par addition d'acide oxalique dans une solution d'un sel soluble de terres rares comme un nitrate de terres rares.

Les composés capables de libérer des ions oxalate ou ammonium peuvent être ajoutés dans le milieu contenant l'oxalate neutre de terres rares sous forme cristallisée ou sous forme de solutions aqueuses.

Ces composés peuvent être ajoutés simultanément ou succesivement, rapidement ou lentement sans influer sur le rendement de la transformation de l'oxalate neutre en oxalate double. Toutefois, la vitesse d'introduction peut avoir une influence sur la granulométrie de l'oxalate double, par exemple sur la répartition granulométrique.

Par ailleurs, les concentrations en ions $(C_2O_4)^=$ et $NH_4^+$ dans les solutions ne sont pas critiques et peuvent varier dans de larges limites.

Les conditions de mise en oeuvre du procédé sont peu critiques pour obtenir un oxalate double. Toutefois, le contrôle de la vitesse de mélanges des différentes solutions ou la vitesse d'introduction des produits cristallisés dans le milieu contenant l'oxalate neutre, de la température, de l'agitation du mélange permet de modifier et contrôler la morphologie de l'oxalate double précipité.

En outre, la température a une influence sur le rendement de la transformation car le coefficient de solubilité de l'oxalate double et de l'oxalate neutre augmente avec l'élévation de la température.

Selon une caractéristique préférentielle de l'invention, le procédé est mis en oeuvre à une température comprise entre 50°C et 90°C, de préférence entre 60°C et 90°C.

Selon une autre caractéristique préférentielle de l'invention, la séparation du précipité est réalisée entre 5 min et 2 H environ après la fin de précipitation. Pendant cette période, le milieu réactionel peut être maintenu agité ou non.

Cette étape permet un réarrangement des cristaux et est généralement appelée une étape de mûrissement du précipité.

Le précipité obtenu est séparé du liquide surnageant par tout procédé de séparation solide/liquide comme par exemple, filtration, centrifugation décantation ou analogue. Il peut également être soumis à un ou plusieurs lavages, pour, par exemple, éliminer les sels solubles.

L'oxalate double de terres rares et d'ammonium put subir un séchage pour évaporer l'eau non lié par exemple par un traitement thermique entre 50°C et 100°C ou un séchage sous pression réduite.

Le procédé de l'invention permet de produire un oxalate double de terres rares et d'ammonium présentant une dimension moyenne de grains comprise entre 5 et 10μm environ, avantageusement comprise entre 7 et 10 μm. Ces grains ont une forme de cube.

Par aileurs, la dimension des grains d'oxalate double de terres rares obtenus est fonction de la dimension des grains d'oxalate neutre utilisé, la dimension de l'oxalate double étant supérieure à celle de l'oxalate neutre de départ.

Une des utilisations de ces oxalates doubles de terres rares et d'ammonium est la production d'oxyde de terres rares obtenu par décomposition thermique de ceux-ci.

La morphologie et granulométrie des oxydes de terres rares obtenus par décomposition d'un oxalate double est généralement semblable à celle dudit oxalate double utilisé comme précurseur. Toutefois, selon les conditions de traitement thermique de l'oxalate double, la granulométrie de l'oxyde peut être légèrement différente de celle de l'oxalate.

Ainsi, les oxydes obtenus par calcination des oxalates doubles de la présente invention ont des dimensions moyennes de grains comprises entre 5 et 10 μm environ, de préférence entre 7 et 10 μm, avec un $\frac{\sigma}{m}$ compris entre 0,35 et 0,6.

Le facteur $\frac{\sigma}{m}$ représente la dispersibilité des tailles de particule ou grain et est calculé par la formule suivante :

$$\frac{\sigma}{m} \quad \frac{\phi_{84} - \phi_{16}}{2\phi_{50}}$$

dans laquelle :

$\phi_{84}$ représente le diamètre de grain pour lequel 84 % des grains ont un diamétre inférieur à $\phi_{84}$.

$\phi_{16}$ représente le diamètre de grain pour lequel 16 % des grains ont un diamétre inférieur à $\phi_{16}$.

$\phi_{50}$ représente le diamètre moyen des grains.

Le traitement thermique ou calcination est générallement réalisée à une température comprise entre 600 et 1200°C, avantageusement entre 800°C et 1000°C.

La durée de calcination est déterminée de manière classique par le contrôle du poids constant. A titre indicatif, la durée de la calcination peut varier entre 30 minutes et 6 heures environ.

L'invention sera illustrée par des exemples donnés ci-dessous uniquement à titre indicatif.

Exemple 1

Une suspension contenant un oxalate neutre d'yttrium préalablement séché à 30°C est portée à 85°C.

Une solution d'oxalate d'ammonium 0,255M est ajoutée pour avoir les rapports d'espèces suivants :

$$C_2O_4^{=}/Y = 2$$

$$NH_4^{+}/Y = 2$$

Le milieu réactionnel est maintenu pendant une heure sous agitation.

Le précipité est récupéré par filtration et lavage à l'eau, puis séché à 100°C.

Sa structure d oxalate double d yttrium et d'ammonium est confirmée par analyse aux rayons X.

Ce sel est ensuite calciné à 900°C pendant une heure.

Les caractéristiques granulométriques de l'oxyde obtenu sont déterminées par analyse avec le granulométre CILAS ®.

L'oxyde d'yttrium présente un diamètre moyen $\phi_{50}$ égal à 5,9 µm et un $\frac{\sigma}{m}$ égal à 0,44.

Exemple 2

On reproduit l'exemple 1 mais avec les rapports d'espéces suivants :

$$C_2O_4^{=}/Y = 2,63$$

$$NH_4^{+}/Y = 4$$

L'oxyde obtenu présente un $\phi_{50}$ égal à 8 µm et un $\frac{\sigma}{m}$ égal à 0,6.

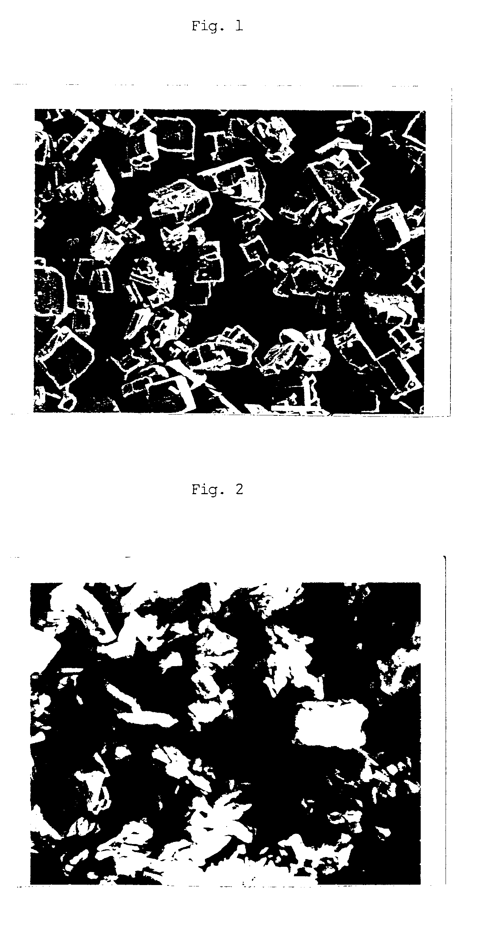

L'oxyde d'yttrium obtenu par calcination de cet oxalate double est illustré à la figure 1 (grossissement 1200 fois).

A titre comparatif, la figure 2 illustre l'oxyde obtenu par calcination de l'oxalate neutre de départ (grossissement 1200 fois).

Exemple 3

Dans une suspension d'oxalate neutre d'yttrium on ajoute de l'acide oxalique pour avoir un rapport $C_2O_4^{=}/Y$ égal à 2,35.

Cette suspension est chauffée à 85°C.

Une solution d'ammoniac 3,1N est ajoutée de maniére à obtenir un rapport $NH_4^{+}/Y$ égal à 1,13.

Le mélange est maintenu sous agitation pendant 30 minutes puis le précipité est filtré et lavé à l'eau.

Après séchage et calcination selon le procédé décrit dans les exemples 1 et 2, l'oxyde obtenu est analysé au granulométre CILAS ®. Il présente un $\phi_{50}$ égal à 7 µm et un $\frac{\sigma}{m}$ égal à 0,43.

**Revendications**

1. Procédé de fabrication d'un oxalate double de terres rares et d'ammonium caractérisé en ce qu'il consiste :

   - à mélanger, en milieu aqueux, au moins un oxalate neutre de terres rares avec un composé capable de libérer des ions ammonium et un composé capable de libérer des ions oxalates en solution
   - à séparer le précipité obtenu
   - et, éventuellement le sécher.

2. Procédé selon la revendication 1, caractérisé en ce que le rapport molaire des ions oxalates aux ions de terres rares est supérieur ou égal à 2 .

3. procédé selon la revendication 1 ou 2 caractérisé en ce que le composé capable de libérer des ions ammonium est choisi parmi le nitrate d'ammonium, le chlorure d'ammonium, l'acétate d'ammonium, l'hydroxyde d'ammonium.

4. Procédé selon l'une des revendications précédentes caractérisé en ce que le composé capable de libérer des ions oxalates est choisi parmi l'acide oxalique cristallisé ou en solution, les oxalate d'alcalins.

5. Procédé selon l'une des revendications 1 ou 2, caractérisé en ce que l'oxalate d'ammonium est utilisé comme composé capable de libérer simultanément des ions ammonium et oxalates .

6. Procédé selon l'une des revendications précédentes, caractérisé en ce que les rapports molaires $C_2O_4^=$/TR et $NH_4^+$/TR à la fin de la précipitation sont supérieurs ou égaux à 2, de préférence supérieurs ou égaux à 2,5.

7. Procédé selon l'une des revendications précédentes, caractérisé en ce que l'oxalate neutre de terre rare est un oxalate d'yttrium, europium, lanthane, néodyme, dysprosium, cérium, gadolinium, terbium ou un mélange de ceux-ci .

8. Procédé selon l'une des revendications précédentes caractérisé en ce que la précipitation est réalisée à une température comprise entre 50°C et 90°C, de préférence entre 60°C et 90°C .

9. Procédé selon l'une des revendications précédentes caractérisé en ce que la séparation du précipité est réalisée entre 5 min et 2 H après la fin de précipitation .

10. Procédé de fabrication d'oxyde de terres rares, caractérisé en ce qu'il consiste à calciner l'oxalate double d'ammonium et de terre rare obtenu selon l'une des revendications précédentes, après un séchage éventuel.

11. Procédé selon la revendication 10 caractérisé en ce que la température de calcination est comprise entre 600 et 1200°C, de préférence entre 800°C et 1000°C.

12. Oxyde de terre rares obtenus selon l'une des revendications 10 ou 11 caractérisé en ce qu'il présente une taille de grains comprises entre 5 et 10 μm, de préférence entre 7 et 10 μm, avec un indice de dispersion $\frac{\sigma}{m}$ compris entre 0,35 et 0,6.

## Claims

1. Process for the manufacture of an ammonium rare-earth double oxalate, characterized in that it consists in:

   - mixing, in an aqueous medium, at least one rare-earth neutral oxalate with a compound capable of liberating ammonium ions and a compound capable of liberating oxalate ions in solution,
   - separating the precipitate obtained,
   - and, where appropriate, drying it.

2. Process according to claim 1, characterized in that the mole ratio of oxalate ions to rare-earth ions is not less than 2.

3. Process according to claim 1 or 2, characterized in that the compound capable of liberating ammonium ions is selected from ammonium nitrate, ammonium chloride, ammonium acetate and ammonium hydroxide.

4. Process according to one of the preceding claims, characterised in that the compound capable of liberating oxalate ions is selected from oxalic acid, crystallized or in solution, and alkali metal oxalates.

5. Process according to one of claims 1 and 2, characterized in that ammonium oxalate is used as a compound capable of simultaneously liberating ammonium and oxalate ions.

6. Process according to one of the preceding claims, characterized in that the $C_2O_4^=$/RE and $NH_4^+$/RE mole ratios at completion of the precipitation are not less than 2, and preferably not less than 2.5.

7. Process according to one of the preceding claims, characterized in that the rare-earth neutral oxalate is an yttrium, europium, lanthanum, neodymium, dysprosium, cerium, gadolinium or terbium oxalate, or a mixture of these.

8. Process according to one of the preceding claims, characterized in that the precipitation is carried out at a temperature of between 50°C and 90°C, and preferably between 60°C and 90°C.

9. Process according to one of the preceding claims, characterized in that the separation of the precipitate is carried out between 5 min and 2 h after completion of precipitation.

10. Process for the manufacture of a rare-earth oxide, characterized in that it consists in calcining the ammonium rare-earth double oxalate obtained according to one of the preceding claims, after drying where appropriate.

11. Process according to claim 10, characterized in that the calcination temperature is between 600 and 1200°C, and preferably between 800°C and 1000°C.

12. Rare-earth oxide obtained according to one of claims 10 and 11, characterized in that it possesses a particle size of between 5 and 10 μm, and preferably between 7 and 10 μm, with a dispersion index $\frac{\sigma}{m}$ of between 0.35 and 0.6.

## Patentansprüche

1. Verfahren zur Herstellung eines Seltenen Erde/Ammonium-Doppeloxalats, dadurch gekennzeichnet, daß das Verfahren darin besteht:

   - in wäßrigem Milieu mindestens ein neutrales Oxalat von Seltenen Erden mit einer Verbindung, die Ammoniumionen freisetzen kann, und einer Verbindung, die Oxalationen in Lösung freisetzen kann, zu vermischen,
   - den erhaltenen Niederschlag abzutrennen und
   - ihn gegebenenfalls zu trocknen.

2. Verfahren nach Anspruch 1, dadurch gekennzeichnet, daß das Molverhältnis von Oxalationen zu Seltenen Erdmetallionen größer oder gleich 2 ist.

3. Verfahren nach Anspruch 1 oder 2, dadurch gekennzeichnet, daß die Verbindung, die Ammoiniumionen freisetzen kann, ausgewählt ist aus Ammoniumnitrat, Ammoniumchlorid, Ammoniumacetat und Ammoniumhydroxid.

4. Verfahren nach einem der vorhergehenen Ansprüche, dadurch gekennzeichnet, daß die Verbindung, die Oxalationen freisetzen kann, ausgewählt ist aus kristallisierter oder in Lösung befindlicher Oxalsäure und Alkalioxalaten.

5. Verfahren nach Anspruch 1 oder 2, dadurch gekennzeichnet, daß das Ammoniumoxalat als die Verbindung verwendet wird, die gleichzeitig Ammonium- und Oxalationen freisetzen kann.

6. Verfahren nach einem der vorhergehenden Ansprüche, dadurch gekennzeichnet, daß die Molverhältnisse von $C_2O_4^{2-}$/Seltene Erde und $NH_4^+$/Seltene Erde am Ende der Fällung größer oder gleich 2, vorzugsweise größer oder gleich 2,5, sind.

7. Verfahren nach einem der vorhergehenden Ansprüche, dadurch gekennzeichnet, daß das neutrale Oxalat der Seltenen Erde ein Yttrium-, Europium-, Lanthan-, Neodym-, Dysprosium-, Cer-, Gadolinium- oder Terbiumoxalat oder eine Mischung dieser Oxalate ist.

8. Verfahren nach einem der vorhergehenden Ansprüche, dadurch gekennzeichnet, daß die Fällung bei einer Temperatur zwischen 50 °C und 90 °C, vorzugsweise zwischen 60 °C und 90 °C, durchgeführt wird.

9. Verfahren nach einem der vorhergehenden Ansprüche, dadurch gekennzeichnet, daß die Abtrennung des Niederschlags 5 Minuten bis 2 Stunden nach Ende der Fällung durchgeführt wird.

10. Verfahren zur Herstellung eines Oxids Seltener Erden, dadurch gekennzeichnet, daß man das Ammonium/Seltene Erde-Doppeloxalat, das nach einem der vorhergehenden Ansprüche erhalten ist, gegebenenfalls nach Trocknung, calciniert.

11. Verfahren nach Anspruch 10, dadurch gekennzeichnet, daß die Calcinierungstemperatur 600 °C bis 1200 °C, vorzugseise 800 °C bis 1000 °C, beträgt.

12. Oxid Seltener Erden, erhalten nach Anspruch 10 oder 11, dadurch gekennzeichnet, daß es eine Korngröße zwischen 5 und 10 $\mu$m, vorzugsweise zwischen 7 und 10 $\mu$m, mit einem Dispersionsindex $\sigma$/m zwischen 0,35 und 0,6 aufweist.

Fig. 1

Fig. 2